# EUROPÄISCHE PATENTSCHRIFT

(11) **EP 4 371 515 B1**
(45) Veröffentlichungstag und Bekanntmachung des Hinweises auf die Patenterteilung: **28.05.2025**
(21) Anmeldenummer: 22208604.3
(22) Anmeldetag: 21.11.2022
(51) Int. Cl.: A61B 18/14, A61B 17/29, A61B 18/00, A61B 90/00

(54) **KOAGULATIONSINSTRUMENT**
COAGULATION INSTRUMENT
INSTRUMENT DE COAGULATION

(43) Veröffentlichungstag der Anmeldung: 22.05.2024
(73) Patentinhaber: Erbe Elektromedizin GmbH, 72072 Tübingen (DE)
(72) Erfinder: SCHOENHAAR, Lorenz, 72654 Neckartenzlingen (DE); KAUPP, Stefan, 72072 Tuebingen (DE); BOB, Felix, 72108 Rottenburg (DE)
(74) Vertreter: Rüger Abel Patentanwälte PartGmbB

(56) Entgegenhaltungen:
- EP-A1- 2 853 219
- US-A1- 2011 072 638
- US-A1- 2013 046 303

## Beschreibung

Die Erfindung betrifft ein Instrument mit mindestens einer mit biologischem Gewebe in Kontakt überführbaren Elektrode zur Bestromung von biologischem Gewebe.

Instrumente der genannten Bauart, insbesondere Koagulationsinstrumente mit Koagulationselektroden, sind prinzipiell bekannt. Die EP 2 853 219 A1 offenbart ein solches Instrument mit einem zangenartigen Werkzeug, das zwei zusammenwirkende, aufeinander zu und voneinander weg bewegbare Branchen aufweist. Jede der beiden Branchen umfasst einen Kunststoffkörper, an dem eine Koagulationselektrode befestigt ist. Die Koagulationselektrode weist einen abgewinkelten Rand auf, der als Verankerungsabschnitt dient und sich in den Kunststoffkörper hinein erstreckt. Zur Verankerung in dem Kunststoff weist der Verankerungsabschnitt Öffnungen auf, die von dem Kunststoff durchgriffen sind. Dadurch wird eine formschlüssige Verankerung der Elektrode an dem Kunststoffkörper bewirkt. Der Kunststoffkörper lässt eine Gewebekontaktfläche frei und weist zentral einen Messerkanal auf, der eine längliche Nut bildet. Diese durchsetzt die Elektrode und erstreckt sich in den Kunststoffkörper hinein.

Die EP 2 719 352 A1 offenbart eine ähnliche Elektrodenanordnung mit einer aus Blech geformten Elektrode, die zu beiden Seiten ihrer Gewebekontaktfläche einen Verankerungsabschnitt aufweist. In diesen sind Verankerungsöffnungen eingebracht, die sich entweder als Durchgangsöffnung durch den Verankerungsabschnitt oder als Taschen in den Verankerungsabschnitt hinein erstrecken. Außerdem offenbart diese Druckschrift auf der Gewebekontaktfläche angeordnete Abstandshalter in Gestalt von elektrisch nichtleitenden, auf der Gewebekontaktfläche angeordneten Erhebungen.

Die US 2021/0205003 A1 offenbart ein Instrument, dessen Branche einen inneren Kunststoffkörper, ein diesen aufnehmenden, im Querschnitt u-förmigen Metallträger und einen äußeren Kunststoffkörper aufweist, wobei an der Oberseite dieser Branche eine Gewebekontaktfläche vorgesehen ist.

Auch die US 2012/01272873 A1 offenbart eine Branche mit einem strukturell aussteifenden Metallinlay und einem inneren, den Messerkanal definierenden Isolator, der von einer Elektrode und deren Verankerungsabschnitt übergriffen ist. Nach außen ist die Branche von einem weiteren Kunststoffkörper umgeben, der den Verankerungsabschnitt der Elektrode ebenso berührt wie den inneren Kunststoffkörper und den aussteifenden Metallkörper.

Weitere aus Kunststoff und Metallteilen aufgebaute Branchen sind aus der DE 10 2016 100 549 A1 sowie der EP 2 092 905 B1 bekannt.

US 2011/072638 A1 offenbart ein Koagulationsinstrument nach dem Oberbegriff des Anspruchs 1.

Instrumente der genannten Bauart existieren als Einweginstrumente sowie als sterilisierbare mehrfach wiederverwendbare Instrumente. Sowohl bei normalem Einsatz als auch bei der Sterilisierung sind die Instrumente thermischen Belastungen ausgesetzt, die, z.B. in Folge unterschiedlicher Ausdehnungskoeffizienten von Kunststoff und Metall, zu Spaltbildung führen können. Dies kann sowohl bei der Herstellung der Keimfreiheit bei der Sterilisation, als auch hinsichtlich der mechanischen Belastbarkeit zu Schwierigkeiten führen. Deswegen ist es sowohl bei Einweginstrumenten als auch bei Mehrweginstrumenten wünschenswert, die mit der thermischen Belastung einhergehenden Probleme zu mindern.

Es ist Aufgabe der Erfindung, ein dahingehend verbessertes Koagulationsinstrument zu schaffen.

Das erfindungsgemäße Koagulationsinstrument weist mindestens eine Branche auf, an der eine Elektrode befestigt ist, die eine Gewebekontaktfläche aufweist. Ein solches Instrument kann zum Beispiel ein monopolares Instrument mit einer einzigen Elektrode, oder auch ein nach Art einer Zange ausgebildetes Element mit zwei Branchen sein. Jede der Branchen kann mindestens eine Elektrode aufweisen. Weitere Instrumentenbauformen sind möglich.

Die Elektrode weist einen Verankerungsabschnitt auf, der mit mindestens einer Verankerungsöffnung versehen ist. Die Verankerungsöffnung kann eine Durchgangsöffnung oder auch eine Sacköffnung, beispielsweise in Gestalt einer flachen Tasche oder dergleichen sein. Die Verankerungsöffnung kann durch Stanzen, Stanzbiegen, Bohren, Lasern, Ätzen, Erodieren, Wasserstrahltrennen und andere Bearbeitungsverfahren erzeugt worden sein. Insbesondere kann die Öffnung einen glatten Rand oder einen Rand mit einer oder mehreren sich von diesem weg erstreckenden Laschen sein.

Erfindungsgemäß ist die Elektrode an einer Isolatorbaugruppe gefasst, zu der wenigstens zwei Kunststoffkörper gehören, zwischen die sich der Verankerungsabschnitt erstreckt. Die beiden Kunststoffkörper sind mit der Elektrode und/oder miteinander formschlüssig verbunden. Dazu weist jeder der Kunststoffkörper mindestens einen, vorzugsweise mehrere Vorsprünge auf, die in die mindestens eine oder mehrere Verankerungsöffnungen der Elektrode greifen, das heißt, sich in diese hinein oder durch diese hindurch erstrecken. Alternativ weist nur einer der beiden Kunststoffkörper Vorsprünge auf, die sich durch die Verankerungsöffnungen hindurch und dabei in den anderen Kunststoffkörper hinein erstrecken, wodurch wiederum eine Verzahnung der beiden Kunststoffkörper miteinander erreicht wird.

Wenn die beiden Kunststoffkörper miteinander auf einer oder mehrerer der genannten Weisen verzahnt sind, ist ein Formschluss zwischen den beiden Kunststoffkörpern gegeben, der für eine gute, dauerhafte und sichere Verbindung der beiden Kunststoffkörper miteinander und somit auch für eine sichere Verankerung der Elektrode an der Isolatorbaugruppe sorgt. So können besondere zuverlässige Koagulationsinstrumente hergestellt werden. Dies unabhängig davon, ob die Kunststoffkörper in der Nähe der, bei der oder innerhalb der Verankerungsöffnung miteinander verzahnt sind.

Der Verankerungsabschnitt der Elektrode umgreift einen inneren Kunststoffkörper und wird von einem äußeren Kunststoffkörper selbst umgriffen. Beide Kunststoffkörper sind auf einer der vorgenannten Weisen miteinander und mit dem Verankerungsabschnitt der Elektrode formschlüssig verbunden. Die sich durch die Verankerungsöffnungen erstreckenden Fortsätze des Kunststoffkörpers weisen dabei eine Länge L auf, die wenigstens so groß sein kann, wie die Dicke D des Verankerungsabschnitts. Es ist aber auch möglich, die Länge L der Fortsätze der Kunststoffkörper geringer zu wählen als die Dicke D des Verankerungsabschnitts, so dass die Fortsätze der beiden Kunststoffkörper an einer Kontaktfläche aneinandergrenzen, die vollständig in der Verankerungsöffnung liegt.

Das vorgestellte Konzept gestattet die Ausbildung der Kunststoffkörper aus unterschiedlichen Kunststoffen auch dann, wenn diese Kunststoffe miteinander keine mechanisch festen Schweißverbindungen ausbilden. Die Fertigung kann im Spritzgussverfahren erfolgen, bei dem in einer entsprechenden Spritzgussform zunächst einer der Kunststoffkörper durch Injektion plastifizierten Kunststoffs erzeugt wird. Dabei werden in dem ersten Kunststoffkörper Verankerungsstrukturen ausgebildet, z.B. in Gestalt von Vertiefungen (Sacköffnungen). Nach zumindest teilweisem Erstarren des ersten Kunststoffkörpers kann dann der andere der beiden Kunststoffkörper durch Injektion plastifizierten Kunststoffs erzeugt werden. Der Kunststoff fließt dann in die Verankerungsstrukturen ein und bildet dadurch eine Verzahnung mit dem ersten Kunststoffkörper und der Elektrode. Insbesondere im Bereich des Verankerungsabschnitts der Elektrode wird eine feste, zuverlässige Verbindung zwischen den Kunststoffkörpern geschaffen.

Weitere Einzelheiten vorteilhafter Ausführungsformen der Erfindung ergeben sich aus der Zeichnung, der zugehörigen Beschreibung oder aus Ansprüchen. In der Zeichnung sind Ausführungsbeispiele der Erfindung veranschaulicht. Es zeigen:
Figur 1 ein für den laparoskopischen Einsatz vorgesehenes Koagulationsinstrument, in perspektivischer Prinzipdarstellung,
Figur 2 einen Werkzeugteil des Instruments nach Figur 1, in perspektivischer Darstellung,
Figur 3 eine Branche des Werkzeugs nach Figur 2, in perspektivischer Darstellung,
Figur 4 bis 7 verschiedene Ansichten einer Elektrode des Werkzeugs nach Figur 2 und 3,
Figur 8 eine Ausführungsform einer in Stanz-Biegetechnik hergestellten Verankerungsöffnung des Verankerungsabschnitts der Elektrode, in ausschnittsweiser Perspektivdarstellung,
Figur 9 eine abgewandelte Ausführungsform einer in Stanz-Biegetechnik hergestellten Verankerungsöffnung des Verankerungsabschnitts einer Elektrode eines erfindungsgemäßen Instruments,
Figur 10 die Branche nach Figur 3 in einer Schnittdarstellung, in einer ersten Ausführungsform,
Figur 11 eine ausschnittsweise Schnittdarstellung der Branche nach Figur 10, in perspektivischer Darstellung,
Figur 12 eine ausschnittsweise Schnittdarstellung einer Branche eines erfindungsgemäßen Instruments, in einer zweiten Ausführungsform,
Figur 13 die Branche ähnlich Figur 10 oder 12, in einer weiteren Ausführungsform,
Figur 14 die Branche ähnlich Figur 12, in einer abgewandelten Ausführungsform, und
Figur 15 bis Figur 19 verschiedene Ausführungsformen der Branchen anhand von Schnittdarstellungen der Kunststoffkörper und des Verankerungsabschnitts der Elektrode, geschnitten in Richtung der Verankerungsöffnungen parallel zu der Gewebekontaktfläche, jeweils in ausschnittsweiser Darstellung.

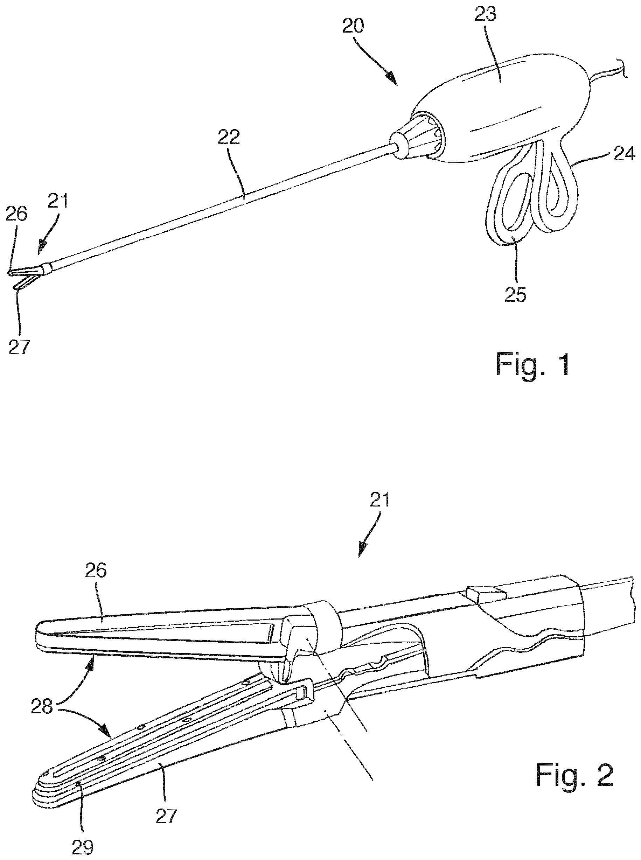

In Figur 1 ist ein Koagulationsinstrument 20 veranschaulicht, das einen zangenartigen Werkzeugteil 21 aufweist. Der Werkzeugteil 21 ist an dem distalen Ende eines Schafts 22 angeordnet, dessen proximales Ende von einem Gehäuse 23 mit einem Handgriff 24 und einem Betätigungshebel 25 getragen ist. Dieser dient der Bewegung von Branchen 26, 27 des Werkzeugteils 21 aufeinander zu und voneinander weg, um dadurch biologisches Gewebe, insbesondere beispielsweise Hohlgefäße, wie Blutgefäße oder dergleichen, zu greifen und zu koagulieren. Dazu sind die beiden Branchen 26, 27 an den aufeinander zu weisenden Seiten jeweils mit einer Elektrode 28 versehen, zwischen denen eine elektrische Spannung angelegt werden kann, so dass ein Strom durch das dazwischen gefasste Gewebe fließt. Der Werkzeugteil 21 kann weitere Elemente, wie z.B. ein mechanisch bewegbares Messer oder eine Schneidelektrode aufweisen.

Das in Figur 1 und 2 dargestellte Instrument 20 ist ein bipolares Werkzeug und soll hier lediglich der Veranschaulichung dienen. Die Erfindung kann jedoch gleichermaßen an Instrumenten Anwendung finden, die für den offenchirurgischen Einsatz oder für den endoskopischen Einsatz ausgebildet sind. Die Erfindung kann auch an Instrumenten Anwendung finden, deren Werkzeugteil 21 lediglich eine einzige Elektrode aufweist und an dem Instrument unbeweglich gehalten ist. Außerdem kann die Erfindung an Instrumenten verwendet werden, die ähnlich Figur 1 und 2 zangenartig ausgebildet sind, bei denen jedoch nur eine der Elektroden beweglich gelagert ist.

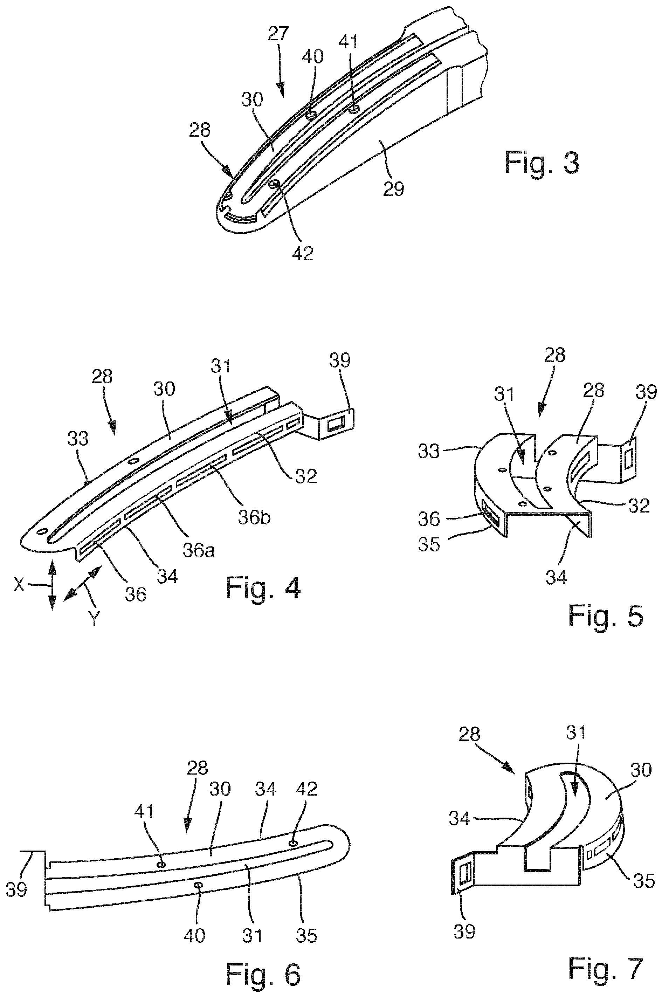

Die Branche 27 ist in Figur 3 gesondert veranschaulicht. Sie umfasst eine Isolatorbaugruppe 29, an der die Elektrode 28 gehalten ist.

Die Elektrode 28 ist in Figur 4, sowie ergänzend in den Figuren 5 bis 7 gesondert veranschaulicht. Sie kann aus einem Blech ausgebildet sein und weist eine Gewebekontaktfläche 30 auf. Die Gewebekontaktfläche 30 kann beispielsweise als ebene Fläche ausgebildet sein. Etwa mittig kann sie von einem sich von dem proximalen Ende zum distalen Ende erstreckenden Messerschlitz 31 durchbrochen sein. Ein solcher Messerschlitz 31 kann vorhanden sein, wenn der Werkzeugteil 21 ein mechanisches, entlang des Messerschlitzes 31 vorschiebbares Messer aufweist (nicht dargestellt).

An den Rand der Gewebekontaktfläche 30 kann sich bei einer Biegelinie 32, 33 ein Verankerungsabschnitt 34, 35 anschließen, der zum Beispiel durch die beiden etwa rechtwinklig nach unten abgewinkelten Ränder der Elektrode 28 gebildet ist. Die Verankerungsabschnitte 34, 35 können an dem distalen Ende der Elektrode 28 ineinander übergehen oder dort, wie in Figur 5 erkennbar, in einem Abstand zueinander enden.

Die Verankerungsabschnitte 34, 35 sind prinzipiell gleich ausgebildet, so dass die nachfolgende Beschreibung des Verankerungsabschnitts 35 für den Verankerungsabschnitt 34 entsprechend gilt. Der Verankerungsabschnitt 35 ist mit mindestens einer, vorzugsweise mehreren Verankerungsöffnungen 36, 36a, 36b versehen, die, wie dargestellt, schlitzartig oder alternativ auch als ovale, rundliche. kreisrunde oder längliche Öffnungen ausgebildet sein können. Diese Öffnungen können prinzipiell als Durchgangsöffnungen oder alternativ auch als flache Taschen ausgebildet sein.

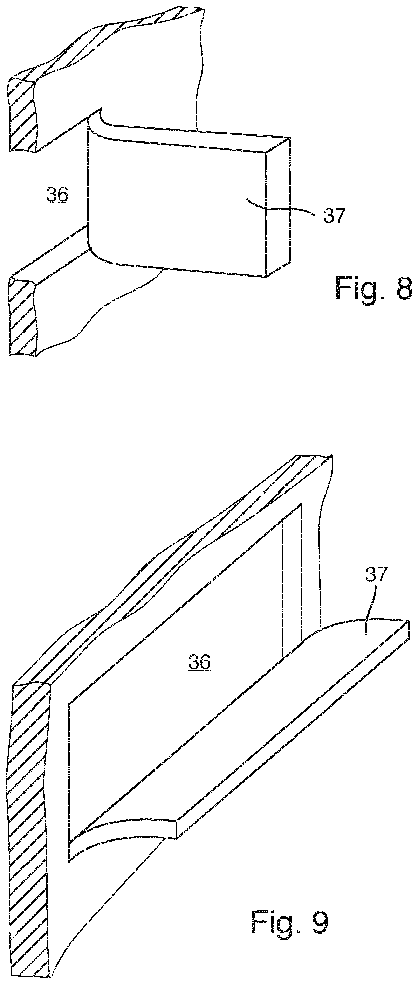

Die Verankerungsöffnungen 36, 36a, 36b können als Öffnungen mit glattem Rand oder auch mit einer randseitig anhängenden Fahne 37, 38 ausgebildet sein, wie es die Figuren 8 oder 9 veranschaulichen. Diese Fahne 37 oder 38 kann beim Erzeugen der Verankerungsöffnung 36 im Stanzverfahren erzeugt werden, indem eine freigestellte Zunge von der Verankerungsöffnung 36 weg gebogen wird. Im Fall der Ausführungsform nach Figur 8 entsteht diese Fahne senkrecht zu der Gewebekontaktfläche 30, während sie bei der Ausführungsform nach Figur 9 im Wesentlichen parallel zu dieser orientiert sein kann. Eine Verankerungsöffnung 36, 36a, 36b kann auch mehrere solcher Fahnen aufweisen.

Die Elektrode 28 ist vorzugsweise als Blechteil ausgebildet und kann gemäß Figur 4 bis 7 eine Kontaktfahne 39 zum Anschluss einer elektrischen Leitung aufweisen. Andere Anschlussmöglichkeiten zum Anschluss eines elektrischen Leiters können ebenso gut vorgesehen sein.

Auf der Gewebekontaktfläche 30 können ein oder mehrere Abstandshalter 40, 41, 42 vorgesehen sein, die vorzugsweise aus einem elektrisch nichtleitenden Material, wie Kunststoff oder Keramik, ausgebildet sind. Die Abstandshalter 40 bis 42 können auch auf die Gewebekontaktfläche 30 aufgebracht sein. Sie können alternativ einen Schaft aufweisen, der durch eine Öffnung der Gewebekontaktfläche 30 sich hindurch erstreckend ausgebildet ist.

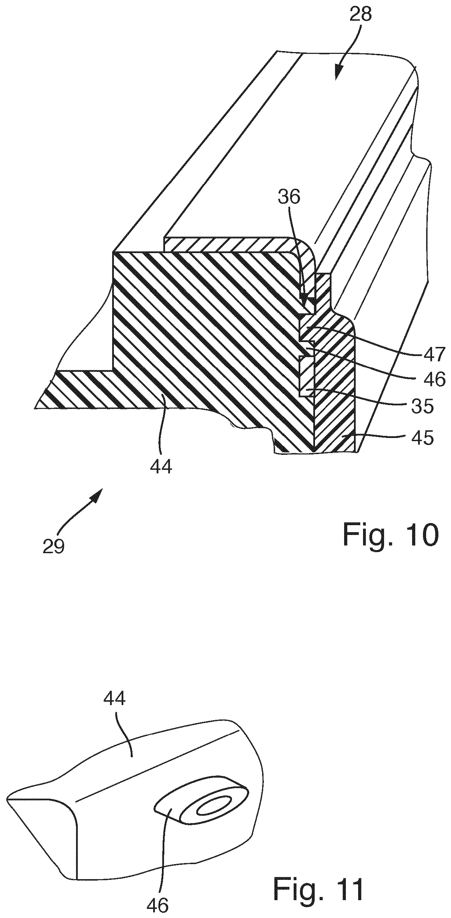

Die insoweit beschriebene Elektrode 28 ist in der Isolatorbaugruppe 29 verankert, die, wie Figur 10 zeigt, einen ersten, inneren, ein- oder mehrteilig ausgebildeten Kunststoffkörper 44 und einen äußeren ein- oder mehrteiligen Kunststoffkörper 45 umfasst, die formschlüssig mit der Elektrode 28 verbunden sind.

Bei dem Ausführungsbeispiel nach Figur 10 sind die beiden Kunststoffkörper 44, 45 im Bereich der Verankerungsöffnung 36 miteinander verzahnt. Dazu weist der innere Kunststoffkörper 44 einen Fortsatz 46 auf, der sich in die Verankerungsöffnung 36 hinein erstreckt. Der zweite Kunststoffkörper 45 weist ebenfalls einen Fortsatz 47 auf, der sich ebenfalls in die Verankerungsöffnung 36 hinein erstreckt. Die beiden Fortsätze 46, 47 können, wie aus Figur 10 ersichtlich, ineinandergreifen, indem beispielweise der erste Fortsatz 46 eine Ausnehmung aufweist, in die der zweite Fortsatz 47 greift. Figur 11 veranschaulicht den Kunststoffkörper 44 und seinen hohlzapfenartigen Fortsatz 46 gesondert. Er weist eine Öffnung auf, in die der zapfenartige Fortsatz 47 des anderen Kunststoffkörpers 45 eingeformt wird.

Durch die so erzielte Verzahnung der Fortsätze 46, 47 der beiden Kunststoffkörper 44, 45 miteinander und somit zugleich die feste Verzahnung mit der Elektrode 28 bzw. dem Verankerungsabschnitt 35 wird eine beständige Verbindung der beiden Kunststoffkörper 44, 45 untereinander erreicht. Dies auch dann, wenn die beiden Kunststoffkörper 44, 45 aus Kunststoffen bestehen, die sich, wenn sie nacheinander in eine Spritzgussform eingebracht werden, nicht stoffschlüssig innig verbinden, beispielsweise miteinander verschweißen. Damit ermöglicht das erfindungsgemäße Konzept die Wahl der Kunststoffe für den ersten und den zweiten Kunststoffkörper 44, 45, ohne dass große Rücksicht auf deren Neigung genommen werden müsste, sich stoffschlüssig zu verbinden. Die Wahl der Kunststoffe kann so hinsichtlich biologischer mechanischer oder elektrischer Eigenschaften sowie thermischer Eigenschaften optimiert werden. Z.B. kann der Kunststoffkörper 44 aus einem verschleißfesten faserverstärkten Kunststoff gefertigt sein, während der Kunststoffkörper 45 aus einem anderen, nicht faserverstärkten Kunststoff besteht. Während der eine Kunststoff hinsichtlich bestimmter Eigenschaft, z.B. Gleiteigenschaften oder Steifigkeit, ausgewählt oder optimiert sein kann, kann der andere Kunststoff hinsichtlich anderer Eigenschaften, z.B. Sterilisierbarkeit oder biologischer Verträglichkeit, ausgewählt oder optimiert sein.

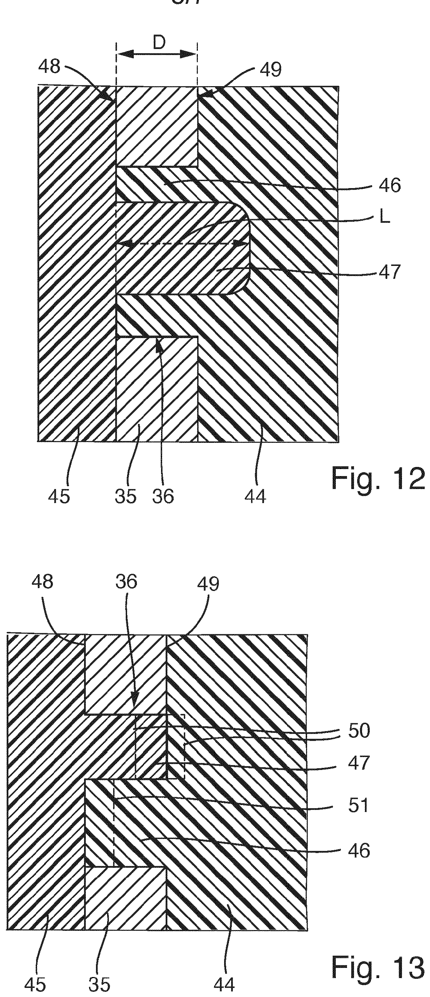

Bei der Ausführungsform nach Figur 10 sind die Fortsätze 46, 47 etwa so lang, dass sie die Verankerungsöffnung 36 füllen, aber nicht durchragen. In Abwandlung davon zeigt Figur 12 eine in dieser Hinsicht modifizierte Ausführungsform in vergrößerter Darstellung. Der an der Außenseite 48 des Verankerungsabschnitts 35 anliegende Kunststoffkörper 45 weist hier einen Fortsatz 47 mit einer Länge L auf, die größer ist, als die zwischen der Außenseite 48 und der Innenseite 49 des Verankerungsabschnitts 35 zu messende Dicke D des Verankerungsabschnitts 35. Während der Fortsatz 47 somit die Innenseite 49 überragt, schließt der Fortsatz 46 mit der Außenseite 48 bündig ab. Die Fortsätze 46, 47 und somit die Kunststoffkörper 44, 45 sind miteinander verzahnt. Außerdem sind sie zusätzlich mit der Elektrode 28 bzw. deren Verankerungsabschnitt 35 verzahnt. Es ist aber auch möglich, die Länge L des Vorsprungs 47 geringer zu bemessen, als die Dicke D.

Bei der Ausführungsform nach Figur 13 weisen die Kunststoffkörper 44, 45 wiederum Fortsätze 46, 47 auf, die sich in die Verankerungsöffnung 36 hinein oder durch diese hindurch erstrecken. Die Fortsätze 46, 47 können sich nebeneinander in die Verankerungsöffnung 36 erstrecken. Wie dargestellt können die Fortsätze 46, 47 mit der Außenseite 48 und der Innenseite 49 bündig abschließen oder diese auch überragen. Auch ist es möglich, den Fortsatz 47 etwas kürzer auszubilden, so dass er von der Außenseite 48 herkommend, die Innenseite 49 nicht erreicht, was in Figur 13 durch gestrichelte Linien 50 angedeutet ist. Zusätzlich oder alternativ kann der in Figur bündig mit der Außenseite 48 dargestellte Fortsatz 46 verkürzt ausgebildet sein und somit innerhalb der Verankerungsöffnung 36 enden, was in Figur 12 durch eine gestrichelte Linie 51 veranschaulicht ist. Dennoch sind, davon unabhängig, die beiden Kunststoffkörper 44, 45 bei der Ausführungsform nach Figur 12 miteinander verzahnt. Sie sind außerdem zugleich mit dem Verankerungsabschnitt 35 der Elektrode 28 verzahnt.

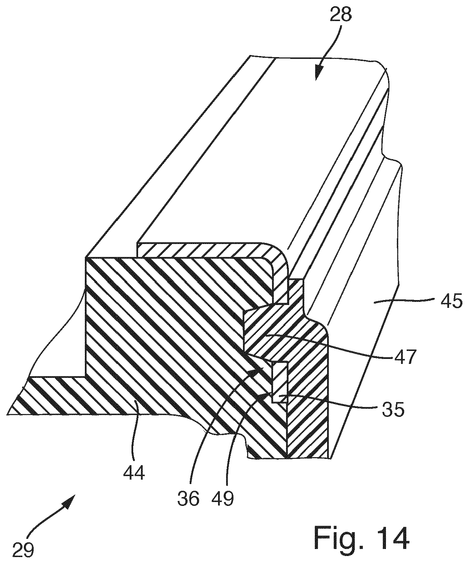

Eine abgewandelte Ausführungsform veranschaulicht Figur 14. Bei dieser durchsetzt der Fortsatz 47 des Kunststoffkörpers 45 die Verankerungsöffnung 36 und erstreckt sich in den Kunststoffkörper 44 hinein. Der Fortsatz 47 erstreckt sich dabei über die Innenseite 49 des Verankerungsabschnitts 35 hinaus in den Kunststoffkörper 44, wodurch die Kunststoffkörper 44, 45 miteinander verzahnt sind. Während der zweite Kunststoffkörper 45 dadurch zugleich mit der Elektrode 28 verzahnt ist, ist der Kunststoffkörper 44 nicht direkt mit der Elektrode 28 verzahnt. Durch die formschlüssige Verbindung der beiden Kunststoffkörper 44, 45 untereinander ist jedoch dennoch eine feste Verbindung zwischen der Elektrode 28 und der Isolatorbaugruppe 29 gegeben.

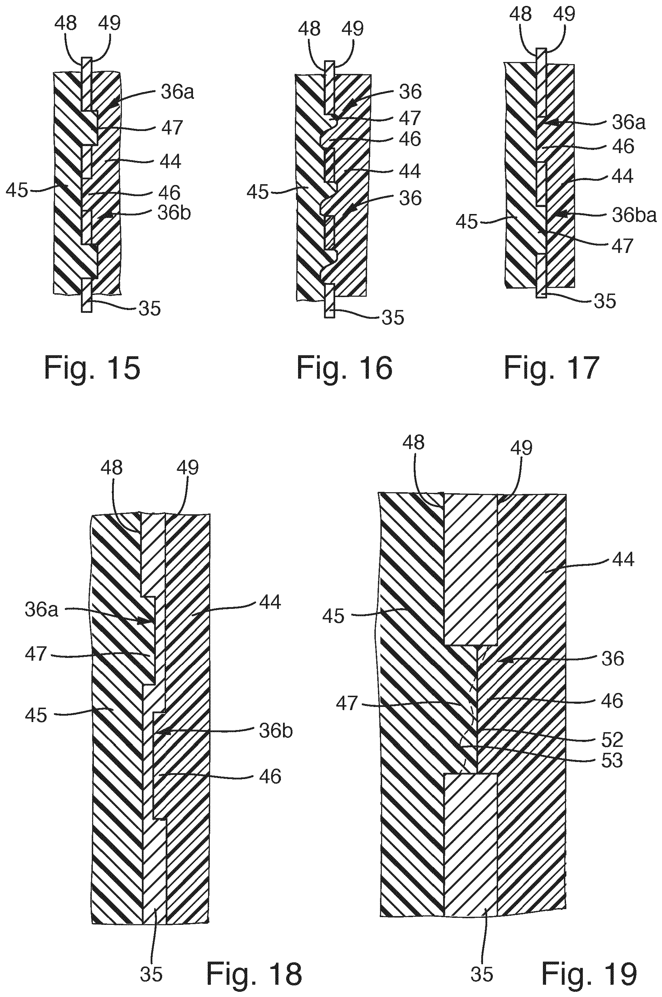

Die Verzahnung der beiden Kunststoffkörper 44, 45 untereinander muss nicht zwangsläufig innerhalb einer einzigen Verankerungsöffnung 36 gegeben sein. Es ist auch möglich, die Verzahnung auf mehrere Verankerungsöffnungen 36, 36a, 36b aufzuteilen, wie es Figur 15 veranschaulicht. Die Verankerungsöffnungen 36a, 36b können, wie es Figur 15 nahelegt, entlang des Verankerungsabschnitts 35 in einer Reihe angeordnet sein. Zum Beispiel kann sich der Fortsatz 47 durch die Verankerungsöffnung 36a nach innen, das heißt über die Innenseite 49 hinaus erstrecken, während sich der Fortsatz 46 von innen nach außen durch die Verankerungsöffnung 36b über die Außenseite 48 hinaus erstreckt. Es ist jedoch auch möglich, dass sich die Fortsätze 46, 47, wie es Figur 16 veranschaulicht, jeweils eine Öffnung 36 teilen und diese nebeneinander durchragen. Sie können dabei bündig mit der Innenseite 49 und der Außenseite 48 abschließen oder auch einzeln oder beide über diese Seiten 48, 49 hinausgehen, wie es Figur 16 veranschaulicht.

In Anlehnung an die Ausführungsform nach Figur 15 ist es auch möglich, die Fortsätze 46, 47 einander abwechselnd in der Reihe der Verankerungsöffnungen 36a, 36b anzuordnen, wobei einer oder beide der Fortsätze 46, 47 jeweils mit den der Außenseite 48 und der Innenseite 49 abschließen. Bei dieser Ausführungsform sind beide Kunststoffkörper 44, 45 mit dem Verankerungsabschnitt 35 der Elektrode 28, nicht aber untereinander verzahnt. Im Gegensatz dazu, sind die Kunststoffkörper 44, 45 bei den Ausführungsformen nach Figur 15 und 16 sowohl miteinander und untereinander, als auch mit dem Verankerungsabschnitt 35 verzahnt.

Figur 18 und Figur 19 veranschaulichen weitere Ausführungsformen, bei denen die Kunststoffkörper 44, 45 mit dem Verankerungsabschnitt 35 und somit der Elektrode 28, jedoch nicht untereinander verzahnt sind. Bei der Ausführungsform nach Figur 18 sind die Verankerungsöffnungen 36a, 36b als flache Taschen ausgebildet, in die sich die Vorsprünge 46, 47 hinein erstrecken. Es wird dadurch eine Verzahnung des Kunststoffkörpers 44 mit dem Verankerungsabschnitt 35 und somit der Elektrode 28 erreicht. Ebenso wird eine Verzahnung des Kunststoffkörpers 45 mit dem Verankerungsabschnitt 35 und somit der Elektrode 28 erreicht.

Bei der Ausführungsform nach Figur 19 wird die Verzahnung der Fortsätze 46, 47 mit dem Verankerungsabschnitt 35 erreicht, in dem sich beide Fortsätze 46, 47 in die Verankerungsöffnung 36 hinein erstrecken und darin treffen. Sie können dabei an einer Fläche 52 zusammentreffen, die sich innerhalb der Verankerungsöffnung 36 befindet. Die Fläche 52 kann eine ebene Fläche oder auch eine nicht ebene, beispielsweise gewölbte Kontaktfläche sein. Sie kann parallel zu der Innenseite 49 und der Außenseite 48 oder auch in einem Winkel zu dieser orientiert sein und sich dabei schräg durch die Verankerungsöffnung 36 erstrecken. Eine solche, nicht ebene und schräg stehende Kontaktlinie ist in Figur 19 als gestrichelte Linie 53 angedeutet.

Ein erfindungsgemäßes Koagulationselement weist eine Isolatorbaugruppe 29 auf, die aus mindestens zwei Kunststoffkörpern 44, 45 besteht, zwischen denen ein Verankerungsabschnitt 35 einer Elektrode 28 angeordnet ist. Der Verankerungsabschnitt 35 weist mindestens eine Verankerungsöffnung, vorzugsweise mehrere Verankerungsöffnungen auf, die zur formschlüssigen Kopplung der Elektrode an die Isolatorbaugruppe 29 dient. Dazu ist mindestens einer der Kunststoffkörper 29, 44 mit einem Fortsatz 47 versehen, der sich in oder durch die Verankerungsöffnung 36 erstreckt. Er kann sich in den anderen Kunststoffkörper 44 hinein erstrecken. Alternativ kann der Kunststoffkörper 44 einen oder mehrere Vorsprünge 46 aufweisen, die sich zumindest in eine Verankerungsöffnung 36 hinein oder durch diese hindurch und gegebenenfalls in den anderen Kunststoffkörper 45 hinein erstrecken.

### Bezugszeichen:

- 20: Koagulationsinstrument
- 21: Werkzeugteil
- 22: Schaft
- 23: Gehäuse
- 24: Griff
- 25: Betätigungshebel
- 26, 27: Branchen
- 28: Elektrode
- 29: Isolatorbaugruppe
- 30: Gewebekontaktfläche
- 31: Messerschlitz
- 32, 33: Biegelinie
- 34, 35: Verankerungsabschnitt
- 36: Verankerungsöffnung 36a, 36b
- 37, 38: Fahne
- 39: Kontaktfahne
- 40 - 42: Abstandshalter
- 44: erster Kunststoffkörper
- 45: zweiter Kunststoffkörper
- 46: Fortsatz des ersten Kunststoffkörpers 44
- 47: Fortsatz des zweiten Kunststoffkörpers 45
- 48: Außenseite des Verankerungsabschnitts 35
- 49: Innenseite des Verankerungsabschnitts 35
- 50, 51: gestrichelte Linie
- 52: Fläche
- 53: gestrichelte Linie

## Patentansprüche

1. Koagulationsinstrument (20), insbesondere zur Koagulation und/oder Fusion von biologischem Gewebe,
mit mindestens einer Branche (26, 27), die eine Elektrode (28) aufweist, die eine Gewebekontaktfläche (30) und mindestens einen Verankerungsabschnitt (35) aufweist, der mindestens eine Verankerungsöffnung (36) aufweist,
**gekennzeichnet durch** eine Isolatorbaugruppe (29), zu der zwei Kunststoffkörper (44, 45) gehören, zwischen die sich der Verankerungsabschnitt (35) erstreckt, wobei beide Kunststoffkörper (44, 45) mit der Elektrode und/oder miteinander formschlüssig verbunden sind.

2. Koagulationsinstrument nach Anspruch 1, **dadurch gekennzeichnet, dass** die Kunststoffkörper (44, 45) miteinander verzahnt sind.

3. Koagulationsinstrument nach einem der vorstehenden Ansprüche, **dadurch gekennzeichnet, dass** die Kunststoffkörper (44, 45) bei der Verankerungsöffnung (36) miteinander verzahnt sind.

4. Koagulationsinstrument nach einem der vorstehenden Ansprüche, **dadurch gekennzeichnet, dass** die Kunststoffkörper (44, 45) innerhalb der Verankerungsöffnung (36) miteinander verzahnt sind.

5. Koagulationsinstrument nach einem der vorstehenden Ansprüche, **dadurch gekennzeichnet, dass** der Verankerungsabschnitt (35) der Elektrode (28) einen der Kunststoffkörper (44) umgreift.

6. Koagulationsinstrument nach einem der vorstehenden Ansprüche, **dadurch gekennzeichnet, dass** der von dem Verankerungsabschnitt (35) umgriffene Kunststoffkörper (44) sich in und/oder durch Öffnungen (36) des Verankerungsabschnitts (35) erstreckende Fortsätze (46) aufweist.

7. Koagulationsinstrument nach einem der vorstehenden Ansprüche, **dadurch gekennzeichnet, dass** einer der Kunststoffkörper (45) den Verankerungsabschnitt (35) der Elektrode (28) umgreift.

8. Koagulationsinstrument nach einem der vorstehenden Ansprüche, **dadurch gekennzeichnet, dass** der Kunststoffkörper (45), der den Verankerungsabschnitt (35) umgreift, sich in und/oder durch Öffnungen (36) des Verankerungsabschnitts (35) erstreckende Fortsätze (47) aufweist.

9. Koagulationsinstrument nach Anspruch 6 oder 8, **dadurch gekennzeichnet, dass** die Fortsätze (46, 47) eine Länge (L) aufweisen, die wenigstens so groß ist, wie die Dicke (D) des Verankerungsabschnitts (36).

10. Koagulationsinstrument nach einem der vorstehenden Ansprüche, **dadurch gekennzeichnet, dass** der Verankerungsabschnitt (35) durch einen sich von der Gewebekontaktfläche (30) abgewinkelt weg erstreckenden Rand gebildet ist, der mit einer Seite (48) mit einem der Kunststoffkörper (45) und mit seiner gegenüberliegenden Seite (49) mit dem anderen der Kunststoffkörper (44) in Berührung steht.

11. Koagulationsinstrument nach einem der vorstehenden Ansprüche, **dadurch gekennzeichnet, dass** die Verankerungsöffnung (36) eine Durchgangsöffnung ist.

12. Koagulationsinstrument nach einem der vorstehenden Ansprüche, **dadurch gekennzeichnet, dass** einer der Kunststoffkörper (44) ein faserverstärkter Kunststoffkörper ist.

13. Koagulationsinstrument nach einem der vorstehenden Ansprüche, **dadurch gekennzeichnet, dass** einer der Kunststoffkörper (44) einen Messerkanal (31) umschließt.

14. Koagulationsinstrument nach einem der vorstehenden Ansprüche, **dadurch gekennzeichnet, dass** die Kunststoffkörper (44, 45) aus unterschiedlichen Kunststoffen ausgebildet sind.

15. Koagulationsinstrument nach einem der vorstehenden Ansprüche, **dadurch gekennzeichnet, dass** die Kunststoffkörper (44, 45) miteinander stoffschlüssig verbunden sind.

## Claims

1. A coagulation instrument (20), in particular for coagulation and/or fusion of biological tissue,
having at least one jaw (26, 27) comprising an electrode (28) comprising a tissue contact surface (30) and at least one anchor section (35) which comprises at least one anchor opening (36),
**characterized by** an insulator unit (29) comprising two plastic bodies (44, 45) between which the anchor section (35) extends, wherein both plastic bodies (44, 45) are connected in form-fitting manner to the electrode and/or to each other.

2. The coagulation instrument according to claim 1, **characterized in that** the plastic bodies (44, 45) are interlocked with one another.

3. The coagulation instrument according to one of the preceding claims, **characterized in that** the plastic bodies (44, 45) are interlocked with one another at the anchor opening (36).

4. The coagulation instrument according to one of the preceding claims, **characterized in that** the plastic bodies (44, 45) are interlocked with one another inside the anchor opening (36).

5. The coagulation instrument according to one of the preceding claims, **characterized in that** the anchor section (35) of the electrode (28) embraces one of the plastic bodies (44, 45).

6. The coagulation instrument according to one of the preceding claims, **characterized in that** the plastic body (44) embraced by the anchor section (35) comprises extensions (46) extending into and/or through openings (36) of the anchor section (35).

7. The coagulation instrument according to one of the preceding claims, **characterized in that** one of the plastic bodies (45) embraces the anchor section (35) of the electrode (28).

8. The coagulation instrument according to one of the preceding claims, **characterized in that** the plastic body (45) embracing the anchor section (35) comprises extensions (47) extending into and/or through openings (36) of the anchor section (35).

9. The coagulation instrument according to claim 6 or 8, **characterized in that** the extensions (46, 47) have a length (L) which is at least as great as the thickness (D) of the anchor section (35).

10. The coagulation instrument according to one of the preceding claims, **characterized in that** the anchor section (35) is formed by an edge extending at an angle away from the tissue contact surface (30), wherein the edge is in contact with one side (48) with one of the plastic bodies (45) and with its opposite side (49) with the other of the plastic bodies (44).

11. The coagulation instrument according to one of the preceding claims, **characterized in that** the anchor opening (36) is a through-opening.

12. The coagulation instrument according to one of the preceding claims, **characterized in that** one of the plastic bodies (44) is a fiber-reinforced plastic body.

13. The coagulation instrument according to one of the preceding claims, **characterized in that** one of the plastic bodies (44) surrounds a knife channel (31).

14. The coagulation instrument according to one of the preceding claims, **characterized in that** the plastic bodies (44, 45) are made of different plastic materials.

15. The coagulation instrument according to one of the preceding claims, **characterized in that** the plastic bodies (44, 45) are connected to one another in a substance bond manner.

## Revendications

1. Instrument de coagulation (20), destiné notamment à la coagulation et/ou à la fusion de tissus biologiques, comprenant au moins un mors (26, 27) qui présente une électrode (28), laquelle comporte une surface de contact de tissu (30) et au moins une partie d'ancrage (35) présentant au moins une ouverture d'ancrage (36),
**caractérisé en ce qu'**il comprend
un module isolateur (29) qui comporte deux corps en matière plastique (44, 45) entre lesquels s'étend la partie d'ancrage (35), les deux corps en matière plastique (44, 45) étant reliés par complémentarité de formes à l'électrode et/ou entre eux.

2. Instrument de coagulation selon la revendication 1, **caractérisé en ce que** les corps en matière plastique (44, 45) engrènent l'un avec l'autre.

3. Instrument de coagulation selon une des revendications précédentes, **caractérisé en ce que** les corps en matière plastique (44, 45) engrènent l'un avec l'autre au niveau de l'ouverture d'ancrage (36).

4. Instrument de coagulation selon une des revendications précédentes, **caractérisé en ce que** les corps en matière plastique (44, 45) engrènent l'un avec l'autre à l'intérieur de l'ouverture d'ancrage (36).

5. Instrument de coagulation selon une des revendications précédentes, **caractérisé en ce que** la partie d'ancrage (35) de l'électrode (28) entoure l'un des corps en matière plastique (44, 45).

6. Instrument de coagulation selon une des revendications précédentes, **caractérisé en ce que** le corps en matière plastique (44) entouré par la partie d'ancrage (35) présente des protubérances (46) qui s'étendent dans et/ou à travers des ouvertures (36) de la partie d'ancrage (35).

7. Instrument de coagulation selon une des revendications précédentes, **caractérisé en ce que** l'un des corps en matière plastique (45) entoure la partie d'ancrage (35) de l'électrode (28).

8. Instrument de coagulation selon une des revendications précédentes, **caractérisé en ce que** le corps en matière plastique (45) qui entoure la partie d'ancrage (35) présente des protubérances (47) qui s'étendent dans et/ou à travers des ouvertures (36) de la partie d'ancrage (35).

9. Instrument de coagulation selon la revendication 6 ou 8, **caractérisé en ce que** les protubérances (46, 47) présentent une longueur (L) qui est au moins aussi grande que l'épaisseur (D) de la partie d'ancrage (35).

10. Instrument de coagulation selon une des revendications précédentes, **caractérisé en ce que** la partie d'ancrage (35) est constituée d'un bord qui s'étend à partir de la surface de contact de tissu (30), en formant un angle, et qui est en contact par un côté (48) avec l'un des corps en matière plastique (45), et par son côté (49) opposé avec l'autre corps en matière plastique (44).

11. Instrument de coagulation selon une des revendications précédentes, **caractérisé en ce que** l'ouverture d'ancrage (36) est une ouverture traversante.

12. Instrument de coagulation selon une des revendications précédentes, **caractérisé en ce que** l'un des corps en matière plastique (44) est un corps en matière plastique renforcée par fibres.

13. Instrument de coagulation selon une des revendications précédentes, **caractérisé en ce que** l'un des corps en matière plastique (44) entoure un conduit de lame (31).

14. Instrument de coagulation selon une des revendications précédentes, **caractérisé en ce que** les corps en matière plastique (44, 45) sont constitués de matières plastiques différentes.

15. Instrument de coagulation selon une des revendications précédentes, **caractérisé en ce que** les corps en matière plastique (44, 45) sont liés l'un à l'autre par matière.
